(19) Europäisches Patentamt
European Patent Office
Office européen des brevets

(11) Publication number: **0 508 242 A2**

# (12) EUROPEAN PATENT APPLICATION

(21) Application number: **92105305.4**

(51) Int. Cl.$^5$: **C12Q 1/32**

(22) Date of filing: **27.03.92**

(30) Priority: **28.03.91 JP 64315/91**

(43) Date of publication of application:
**14.10.92 Bulletin 92/42**

(84) Designated Contracting States:
**DE ES FR GB IT**

(71) Applicant: **CIBA CORNING DIAGNOSTICS K.K.**
**Unosawa Tokyu Bldg., 1-19-15, Ebisu**
**Shibuya-ku, Tokyo(JP)**
Applicant: **SAPPORO IMMUNO DIAGNOSTIC LABORATORY**
**12-20, Shinkawanijo 2-chome, Kita-ku**
**Sapporo-shi, Hokkaido(JP)**
Applicant: **CENTRAL GLASS COMPANY, LIMITED**
**7-1, Kandanishiki-cho 3-chome, Chiyoda-ku**
**Tokyo(JP)**

(72) Inventor: **Naruse, Hiroshi**
**1-14-4, Nishitsutsujigaoka**
**Chofu-shi, Tokyo(JP)**
Inventor: **Tsuji, Akio**
**2-11-7-103, Uehara, Shibuya-ku**
**Tokyo(JP)**

(74) Representative: **Hansen, Bernd, Dr. Dipl.-Chem. et al**
**Hoffmann, Eitle & Partner Patent- und Rechtsanwälte Arabellastrasse 4 Postfach 81 04 20**
**W-8000 München 81(DE)**

## (54) Enzyme reagent, kit, and method for determination of blood phenylalanine.

(57) An enzyme reagent, a kit, and a method for determining phenylalanine in blood with use of a dried blood spot and a microwell used in mass-screening of newborns or infants are disclosed. The enzyme reagent comprises phenylalanine dehydrogenase; the kit comprises said enzyme reagent, said microwell, a reagent for extraction of blood components, and a reagent for quantification of phenylalanine; and the method comprises specifically extracting phenylalanine from blood and determining the phenylalanine with use of said enzyme reagent.

EP 0 508 242 A2

This invention relates to an enzyme reagent, a kit, and a method for determination of phenylalanine (hereinafter abbreviated as Phe) in blood. More particularly, it relates to an enzyme reagent comprising phenylalanine dehydrogenase (hereinafter abbreviated as PheDH), which is for the determination of Phe in dried blood spots on filter paper using a microwell commonly used in mass-screening of newborns or infants; to a kit for blood Phe determination comprising the enzyme reagent, the above-described microwell, a reagent for blood component extraction, and a reagent for Phe quantification; and to a method for determining blood Phe comprising specifically extracting Phe from blood and determining the blood Phe by using the above-described enzyme reagent.

Mass-screening of newborns for early diagnosis by metabolic disorders has been carried out by a so-called Guthrie test in which dried blood spots on prescribed filter paper cards, taken from newborns on the fifth to seventh postnatal day by puncture of the heel with a lancet are sent to an examination center. By this test, sugars and amino acids in the blood sample are determined for diagnosis of galactosemia, maple syrup urine disease, homocysteinuria, etc.

In the Guthrie test, a blood disc 3 to 10 mm in diameter punched out from each dried blood spot on filter paper is subjected to a bacterial inhibition assay (hereinafter abbreviated as BIA method). Determination of Phe by BIA method is carried out by placing the blood disc on a minimum nutritious plate culture medium containing Bacillus subtilis and β-2-thienylalanine, a metabolic antagonist. Phe concentration is determined semiquantitatively from the growth band of the bacterium. The currently spread BIA method is superior in making mass-screening feasible. However, this assay method has disadvantages like the growth band is often unclear, judgement depends on the individual analyst, and test results tend to be unreliable and are not recordable. Further, the result is susceptible to influences of a difference among reagent lots, the conditions for medium preparation, and the like, and skillfullness is required to maintain the optimum conditions and to make certain judgements of abnormal samples. Furthermore, the operation and assaying require much time and labor. Taking into consideration the importance of objectiveness of the test result, it has therefore been demanded to develop a method having high reliability and record preservability as well as a capacity to deal with a large number of samples.

In order to overcome the disadvantages associated with the BIA method, micro fluorometric determination (MFL method) using a fluorometric microplate reader has recently been developed and put to practical use in some screening programs. Although the MFL method is free from the subjectivity of analysis with the naked eye and non-preservability of the test result, attending on the BIA method, it still involves problems in the preparation of components constituting the reagents for determination, maintenance of optimum conditions, and quantitativeness of Phe determination. That is, according to the MFL method, though satisfactory in recordability, capacity of dealing with a large number of samples, and reliability, Phe is determined through measurement of fluorescence intensity of the reaction product but not of Phe per se.

An object of the present invention is to provide an enzyme reagent for determining blood Phe, a kit including the enzyme reagent, and a method for determining blood Phe using the enzyme reagent, with or by which a large number of samples can be assayed in a short time with ease, and blood Phe can be quantitatively determined with high sensitivity and reliability.

The above object of the present invention is accomplished by using an enzyme reagent comprising PheDH.

Figure 1 is a graph of Phe concentration vs. fluorescence intensity obtained in Example 1, indicating that the detection limit of Phe in one dried blood disk of 3 mm in diameter is 0.2 mg/dℓ.

Figure 2 is a graph showing correlation between the results of Example 1 and those obtained by HPLC method.

Figure 3 is a graph of Phe concentration vs. absorbance obtained in Example 2, indicating the detection limit of Phe in one dried blood disc of 3 mm in diameter is 0.5 mg/dℓ.

PheDH which can be used in the present invention can be prepared from various microorganism strains. In particular, PheDH species having less reactivity with L-tyrosine or a branched chain amino acid as a substrate are preferable. For example, Bacillus sphaericus SCRC-R79a (FERM No. 8179), Bacillus badius IAM 11059 (FERM No. 8529), and Sporosarcina ureae SCRC-R04 (FERM No. 8178) are suitable as PheDH-producing strains.

The mechanism of reaction of PheDH is as follows.

$$\text{Phe} + NAD^{+} + H_2O \underset{}{\overset{\text{PheDH}}{\rightleftharpoons}} \text{Phenyl pyruvate} + H^{+} + NH_3 + NADH$$

The reagent kit for Phe determination consists of the above-described enzyme reagent and the following extraction reagent and quantification reagent.

The extraction reagent is for deproteination or immobilization of proteins in blood, 2 to 20% trichloroacetic acid, 0.3 to 0.6M perchloric acid, 5 to 20% sulfosalicylic acid, 5 to 20% sodium tungstate, or a mixture of an organic solvent and water, e.g., diethyl ether-water-acetone-alcohol and methylene chloride-water-acetone-alcohol. Particularly 5% trichloroacetic acid is preferable.

According to the present invention, Phe concentration can be quantitatively determined at high sensitivity by direct measurement of NADH produced through the above-illustrated reaction using a fluorophotometer or a spectrophotometer. Furthermore the reliable results are obtained by addition of a 0.1 to 1% Triton X-100 solution or a buffer at pH 9 to 12 as a quantification reagent. For promoting convenience or obtaining higher sensitivity, fluorometry or colorimetry can be exploited. More specifically, $H_2O_2$ generated from NADH in the presence of 1-methoxy-PMS or diaphorase as an electron carrier is utilized for inducing a reaction using 3-(hydroxyphenyl)-propionic acid and peroxidase in fluorometry or for inducing a reaction using titanium (IV) and 4-(2-pyridylazo)-resorcinol in colorimetry. More convenient colorimetry includes a method using tetrazolium salts as a reduction indicator or chelate colorimetry using reduction-oxidation reaction of cobalt or copper, which are also advantageous in that a cheap spectrophotometer for microplates can be utilized.

Phe determination according to the present invention is carried out as follows.

1. A disc sample (3 mm to 10 mm in diameter) is punched out from a dried blood spot on filter paper.
2. The disc sample is put in a 100 to 400 $\mu\ell$-volume microwell.
3. 10 to 100 $\mu\ell$ of extraction reagent is added to the well.
4. A given time later, the supernatant of the well is transferred to another microwell.
5. Enzyme reagent is added to the well. The reaction mixture is incubated at a given temperature for a given reaction time.
6. A quantification reagent is then added thereto, followed by allowing the mixture to react at a given temperature for a given time.
7. A measurement is made with a fluorophotometer or a spectrophotometer, and a result is calculated.

The microwells used in the present invention are for the extraction of Phe and for the subsequent enzymatic dehydrogenation of Phe. Commercially available various 96-well microplates and separation type microplates are useful. A transfer plate (manufactured by Dynatec Laboratories, Inc.) having a hole of about 1 mm in diameter at the bottom of each well or a stripped cell may also be used for simplification of the extraction.

The present invention further relates to a kit for Phe determination comprising the above-described reagent in combination with a microplate or a stripped cell.

The reagents, kit, and method according to the present invention enable us to conduct operations (inclusive of pretreatment, reacting, washing, and measuring) on a large number of samples simultaneously and in a short assay time with ease owing to the use of a microplate. Each reagent of a kit is well quality controlled by the maker, analysts are objective to the determination results under the optimal conditions. The determination results can be recorded by the use of a fluorophotometer or a spectrophotometer for microplates. Further, with Phe specific PheDH being included in the reagent, and with the reaction of this enzyme being included in the determination procedure, the kit or the method of the present invention makes it possible to determine Phe more accurately.

The present invention is now illustrated in greater detail with reference to Examples, but it should be understood that the present invention is not construed as being limited thereto.

## EXAMPLE 1

(1) Kit Components:

(1-1) Extraction Reagent

5% Trichloroacetic acid solution

(1-2) Enzyme Reagent

0.2 M Glycine.KC$\ell$-KOH buffer

2.5 mM $NAD^+$

0.15 U/m$\ell$ PheDH of Bacillus badius IAM 11059 (FERM-P No. 8529) origin

(2) Microwell:

U-Bottomed microplate (produced by Sanko Plastic K.K.) 8-Well separation type microplate (produced by Labo System K.K.)

(3) Procedure:

Blood discs 3 mm in diameter punched out from each dried blood spot standards and samples were put into each well. 50 μℓ of 5% trichloroacetic acid was added, followed by shaking for 10 minutes. A 40 μℓ portion of the extract was transferred to a well for the next reaction. To each well was added 160 μℓ of the enzyme reagent, and the reaction mixture was incubated at room temperature for 60 minutes. Fluorescence intensity was measured by a microplate fluorophotometer at an excitation wavelength of 360 nm and an emission wavelength of 450 nm. The Phe concentration in the sample was calculated from the standard curve.

(4) Results:

(4-1) Sensitivity

Phe concentrations as low as 0.2 mg/dℓ were measurable by analysis of a dried blood disc of 3 mm in diameter (see Fig. 1).

(4-2) Reproducibility

Reproducibility of the test results on dried blood spots having a Phe concentration of 1.5 mg/dℓ, 7.3 mg/dℓ, and 29.4 mg/dℓ was 8.4%, 4.5%, and 2.8%, respectively.

(4-3) Results of Screening of Newborns

29 samples taken from normal newborns gave an average Phe concentration of 0.85 mg/dℓ - (minimum: 0.24 mg/dℓ; maximum: 1.92 mg/dℓ).

(4-4) Correlation to HPLC

In comparison with the HPLC method using the same dried blood spots, there was obtained a satisfactory correlation as follows:

Coefficent of Correlation (r) = 0.995 (n = 25)
Y (method of invention) = 1.12 × (HPLC method) + 0.283

(see Fig. 2)

## EXAMPLE 2

(1) Kit Components:

(1-1) Extraction Reagent

5% Trichloroacetic acid solution

(1-2) Enzyme Reagent

0.17 M Glycine.KCℓ-KOH buffer
2.5 mM $NAD^+$
0.125 U/mℓ PheDH of Bacillus badius IAM 11059 (FERM-P No. 8529) origin

(1-3) Quantification Reagent

1 mM Co (III)
0.5 mM 2-(5-Bromo-2-pyridylazo)-5-(N-propyl-N-sulfopropylamino)phenol disodium salt
0.05 mM 1-Methoxy-5-methylphenadinium methylsulfate
0.2 M Phosphate buffer

(1-4) Reaction Stopper

0.1 M EDTA

(2) Microwell:

U-Bottomed microplate (produced by Sanko Plastic K.K.) 96-Well separation type microplate (produced by Sumitomo Bakelite Co., Ltd.)

(3) Procedure:

Blood discs 3 mm in diameter punched out from each dried blood spot standards and samples were put into each well. 50 μℓ of 5% trichloroacetic acid was added, followed by shaking for 10 minutes. A 40 μℓ portion of the extract was transferred to a well for the next reaction. To each well was added 160 μℓ of the enzyme reagent, and the mixture was incubated at room temperature for 60 minutes. To the reaction mixture was added 50 μℓ of the quantification reagent, followed by allowing it to stand for 5 minutes. Then, 50 μℓ of the reaction stopper was added thereto. Absorbance was measured with a microplate spectrophotometer at a wavelength of 585 nm. The Phe concentration in the sample was calculated from the standard curve.

(4) Results:

(4-1) Sensitivity

Phe concentrations as low as 0.5 mg/dℓ were measurable by analysis of a dried blood disc of 3 mm in diameter (see Fig. 3).

(4-2) Reproducibility

Reproducibility of the test results on dried blood spots having a Phe concentration of 2.1 mg/dℓ, 6.9 mg/dℓ, and 21.5 mg/dℓ was 9.5%, 6.1%, and 5.2%, respectively.

(4-3) Results of Screening of Newborns

40 samples taken from normal newborns gave an average Phe concentration of 0.97 mg/dℓ - (minimum: 0.50 mg/dℓ; maximum: 1.50 mg/dℓ).

While the invention has been described in detail and with reference to specific examples thereof, it will be apparent to one skilled in the art that various changes and modifications can be made therein without departing from the spirit and scope thereof.

## Claims

1. An enzyme reagent for determination of phenylalanine in blood, comprising phenylalanine dehydrogenase.

2. A kit for determination of phenylalanine in blood, comprising (a) a microwell, (b) a reagent for extraction of blood components, (c) an enzyme reagent as claimed in Claim 1, and (d) a reagent for quantification of phenylalanine.

3. A kit as claimed in Claim 2, wherein said microwell is a microplate, a transfer plate, or a stripped cell.

4. A method for determining phenylalanine in blood, comprising (1) extracting phenylalanine from blood, (2) decomposing phenylalanine in the extract with phenylalanine dehydrogenase, (3) inducing a fluorescence reaction or a colorimetric reaction, and (4) determining a phenylalanine concentration with a fluorophotometer or a spectrophotometer.

5. A method as claimed in Claim 4, wherein a microplate or a stripped cell and a fluorophotometer or spectrophotometer for a microplate are used.

6. A method as claimed in Claim 4, wherein a dried blood spot on filter paper is used as a specimen.

Fig. 1

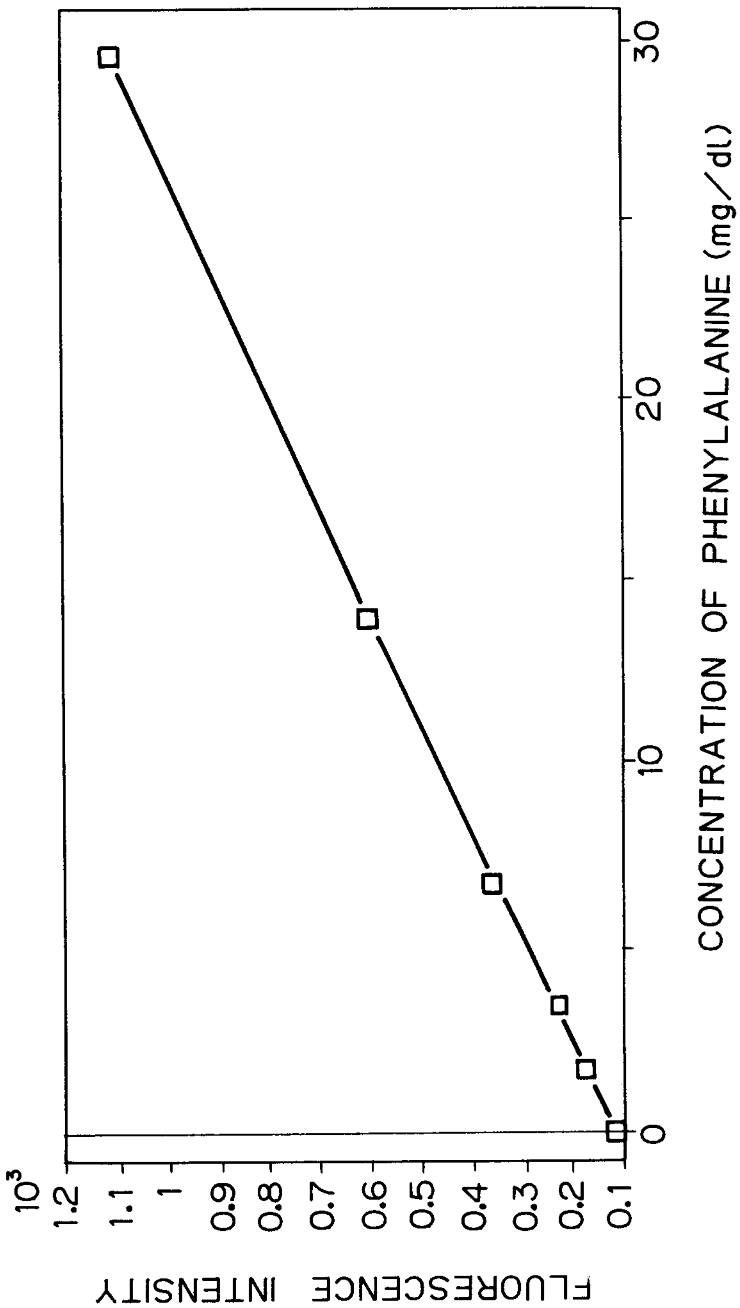
FLUORESCENCE INTENSITY
10³
1.2
1.1
1
0.9
0.8
0.7
0.6
0.5
0.4
0.3
0.2
0.1
0
10
20
30
CONCENTRATION OF PHENYLALANINE (mg/dl)

Fig. 2

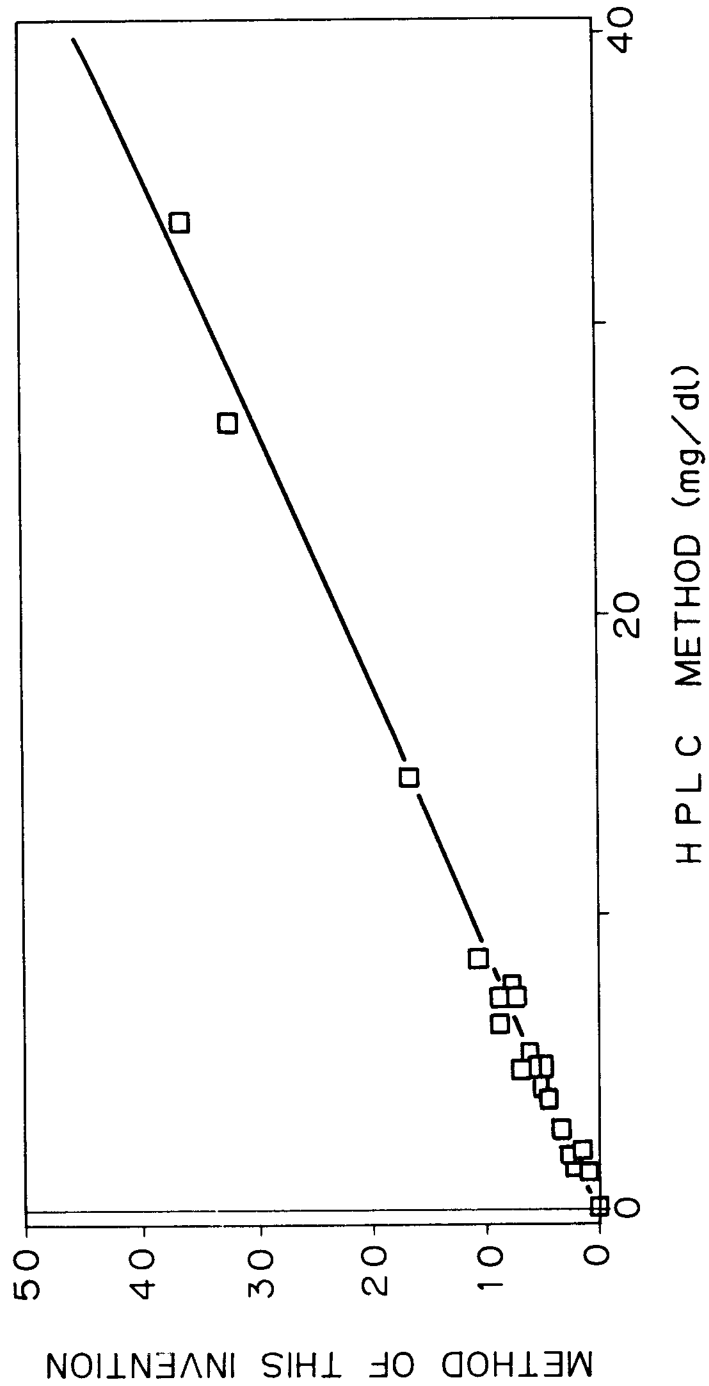
HPLC METHOD (mg/dl)
METHOD OF THIS INVENTION
0
20
40
0
10
20
30
40
50

Fig. 3

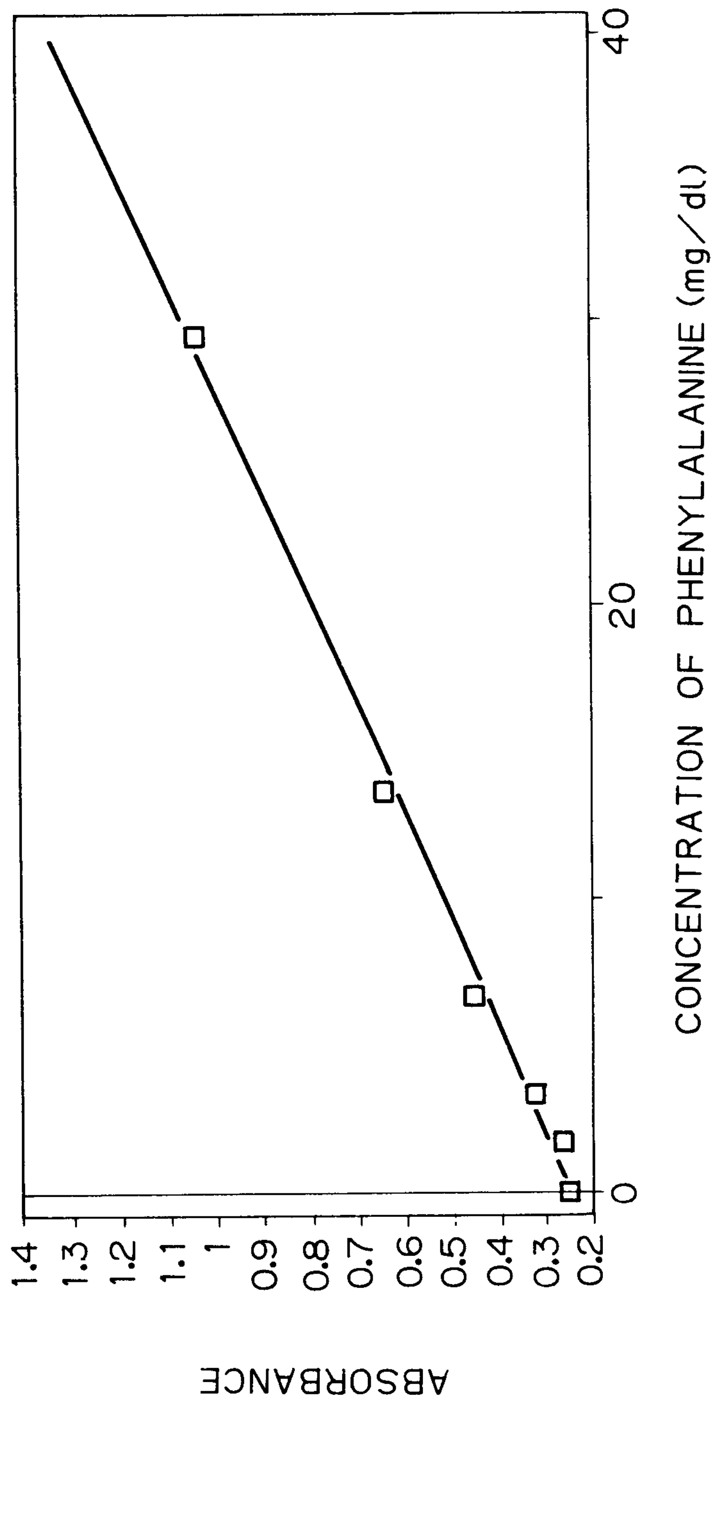
ABSORBANCE
1.4
1.3
1.2
1.1
1
0.9
0.8
0.7
0.6
0.5
0.4
0.3
0.2
0
20
40
CONCENTRATION OF PHENYLALANINE (mg/dl)